# EUROPEAN PATENT SPECIFICATION

(11) **EP 0 796 624 B1**
(45) Date of publication and mention of the grant of the patent: **27.02.2002**
(21) Application number: 97301886.4
(22) Date of filing: 20.03.1997
(51) Int. Cl.: A61K 47/48

(54) **Clathrates and edible compositions comprising the same**
Clathraten und essbare Zusammenzetzungen die diese enthalten
Clathrates et leur compositions comestibles

(30) Priority: 21.03.1996 JP 6437296
(43) Date of publication of application: 24.09.1997
(73) Proprietor: Fujicco Co.,Ltd., Kobe-shi, Hyogo 650 (KP)
(72) Inventor: Kanaoka, Satomi, Osaka-shi, Osaka (JP); Uesugi, Takehiko, Hyogo 663 (JP); Hirai, Kuniaki, Hyogo 674 (JP); Toda, Toshiya, Hyogo 662 (JP); Okuhira, Takenori, Hyogo 651-11 (JP)
(74) Representative: Pett, Christopher Phineas

(56) References cited:
- WO-A-94/15970
- FR-A- 2 542 318
- US-A- 4 826 963
- CHEMICAL ABSTRACTS, vol. 125, no. 9, 26 August 1996 Columbus, Ohio, US; abstract no. 104416, TEICHER, BEVERLY A. ET AL: "Comparison of several antiangiogenic regimens alone and with cytotoxic therapies in the Lewis lung carcinoma" XP002087762 & CANCER CHEMOTHER. PHARMACOL. (1996), 38(2), 169-177 CODEN: CCPHDZ;ISSN: 0344-5704,1996,

## Description

The present invention relates to clathrates with cyclodextrin of isoflavone derivatives contained in soybean or fermented soybean (the Japanese food "*natto*"), and edible compositions comprising the clathrates. More particularly, the present invention relates to clathrates of isoflavone derivatives wherein the bitterness, acerbity and astringency originally expressed by the isoflavone derivatives are suppressed, and the water solubility of the isoflavone derivatives is improved. Thus the isoflavone derivatives can be suitably utilized for edible compositions. Moreover, the functions in a living body of those isoflavone derivatives are improved through an increase in the absorption rate into a human body of the isoflavone derivatives.

Isoflavone derivatives are one of the flavonoid glycosides contained in plants such as *Glycine,* etc.. Physiological activity of the isoflavone derivatives are known in general, such as estrogen action, anti-oxidative action, and antimicrobial action.

It is well known that the mortality of Japanese people resulting from breast cancer or prostate cancer is considerably lower than Western people. Epidemiological investigation has confirmed that the urinary excretion of daidzin in Japanese females is ten times larger than that of females in Boston or Helsinki, and that isoflavone levels in the blood of Japanese males is much higher than that of Finnish males, as high as 40 times higher.

Accordingly, it has been reported that the mortality of the Japanese resulting from breast cancer or prostate cancer has been kept at a low level because the Japanese ingest much larger amounts of isoflavone derivatives. [See, H.Adlercreutz, H.Honjo, A.Higashi, T.Fotsis, E.Hamalaimen, T.Hasegawa and H.Okada Am. J. Clin. Nutr., 54,1093(1991); H.Adlercreutz, H.Markkanen and S.Watanabe: Lancet, 342,1209(1993)]. Consequently, carcinostatic actions by the isoflavone derivatives have been suggested.

Such high isoflavone derivative levels in urine or blood of the Japanese people is presumed to result from the Japanese traditional habit of taking a lot of soybean or food made of/from soybean.

Moreover, it was confirmed that isoflavone derivatives suppress decrease in bone density in ovariectomised rats with a low calcium diet. Thus the isoflavone derivatives are suggested as prophylactic compounds for osteoporosis (See Japanese Patent Provisional Publication No.7-36598).

As mentioned above, isoflavone derivatives have various useful functions, and it is believed that taking isoflavone derivatives would play an important role in keeping and promoting good health.

Isoflavone derivatives may be extracted from soybean or fermented soybean using a solvent such as hot water, alcohol or the like. The majority of the isoflavone derivatives thus extracted are daidzin and genistin. Threshold values of daidzin, genistin and glycitin are 10⁻² mM, 10⁻¹ mM and 10⁻³ mM, respectively. Those values for the malonylated form, acetylated form or aglycon thereof tend to be even lower [See Kazuyoshi Okubo, Yumiko Yoshiki, Masaki Yoshikoshi, Chigen Tsukamoto, Shigemitsu Kudo: New Food Industry, 36(1994)].

Besides having such low threshold values, i.e., bitterness, acerbity and astringency, the isoflavone derivatives are slightly soluble in water. Therefore, application of the isoflavone derivatives to edible compositions containing them has not been carried out in view of such grounds.

In addition, during the steps of making processed foods which are essentially derived from soybean, e.g., soymilk, tofu (soybean curd) and the like, the isoflavone derivatives which are effective in maintaining good health as above described have often been intentionally removed in order to avoid the characteristic taste of the isoflavone derivatives.

Furthermore, because the low water solubility of the isoflavone derivatives leads to low absorption rates into the human body, sufficient beneficial effect cannot be achieved unless large amounts of the derivatives are given.

As stated above, although the isoflavone derivatives have remarkable effects, they have not been utilized in edible compositions because of flavour problems and their low water solubility.

US Patent 4,826,963 describes complexes containing 7-isopropoxy-isoflavone and cyclodextrins. US Patent 4,557,927 relates to alpha-glycosylation of soybean glycosides to improve taste. WO Patent 94/15970 describes cyclodextrin derivatives and their binding to aflotoxins and phytoestrogens.

We have now found a way to enable the positive application of the isoflavone derivatives to edible compositions, and to improve their physiological actions in the living body through raising the absorption rate into the body by suppressing the bitterness, acerbity and astringency thereof, and improving the water solubility of the isoflavone derivatives.

The inventors made strenuous efforts and accomplished the present invention through finding that the bitterness, acerbity and astringency of the isoflavone derivatives can be suppressed and water solubility thereof can be improved by clathrating the isoflavone derivatives with cyclodextrins.

Thus, the present invention is directed to clathrates of isoflavone derivatives. Herein said isoflavone derivatives are clathrated with β-cyclodextrin and/or γ-cyclodextrin.

Figure 1 is a graph illustrating formation of the clathrate of daidzin with various cyclodextrins.

Figure 2 is a graph illustrating formation of the clathrate of genistin with various cyclodextrins.

Figure 3 is a graph illustrating formation of the clathrate of daidzein with various cyclodextrins.

Figure 4 is a graph illustrating formation of the clathrate of genistein with various cyclodextrins.

Figure 5 is a graph illustrating formation of the clathrate of 6"-O-malonyldaidzin, 6"-O-malonylgenistin or 6"-O-malonylglycitin with various cyclodextrins.

Figure 6 is a graph illustrating formation of the clathrate of 6"-O-acetyldaidzin, 6"-O-acetylgenistin or 6"-O-acetylglycitin with various cyclodextrins.

Figure 7 is a graph illustrating formation of the clathrate of 6"-O-succinyldaidzin, 6"-O-succinylgenistin or 6"-O-succinylglycitin with various cyclodextrins.

Figure 8 is a graph illustrating formation of the clathrate of glycitin or glycitein with various cyclodextrins.

Fifteen compounds have been found and identified heretofore as isoflavone derivatives contained in soybean and natto (fermented soybean). Each of these isoflavone derivatives has a bitter, acerbic and astringent taste, and irritation will be induced in one's throat for a while after they are orally taken.

The names of the compounds and structures of those fifteen isoflavone derivatives are listed in Table 1 and Table 2 with reference to the general formulae [I] and [II] respectively.

**Table 1**

| isoflavone derivatives having the general formula [I] | | R¹ | R² | R³ |
|---|---|---|---|---|
| 1. | daidzin | H | H | H |
| 2. | glycitin | H | OCH₃ | H |
| 3. | genistin | H | H | OH |
| 4. | 6''-O-acetyldaidzin | COCH₃ | H | H |
| 5. | 6''-O-acetylglycitin | COCH₃ | OCH₃ | H |
| 6. | 6''-O-acetylgenistin | COCH₃ | H | OH |
| 7. | 6''-O-malonyldaidzin | COCH₂COOH | H | H |
| 8. | 6''-O-malonylglycitin | COCH₂COOH | OCH₃ | H |
| 9. | 6''-O-malonylgenistin | COCH₂COOH | H | OH |
| 10. | 6''-O-succinyldaidzin | COCH₂CH₂COOH | H | H |
| 11. | 6''-O-succinylglycitin | COCH₂CH₂COOH | OCH₃ | H |
| 12. | 6''-O-succinylgenistin | COCH₂CH₂COOH | H | OH |

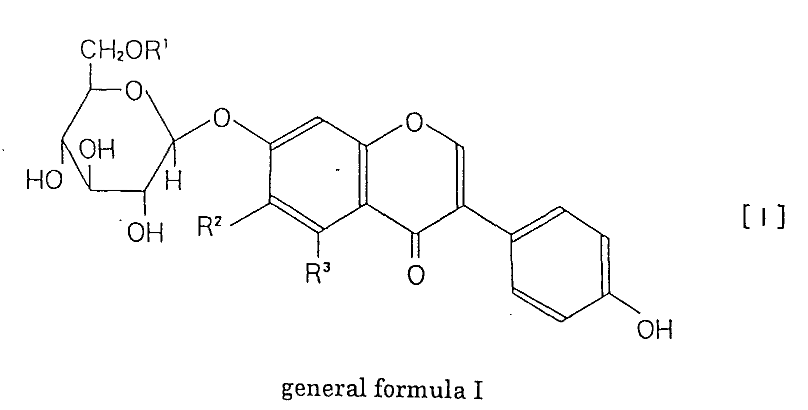

**Table 2**

| isoflavone derivatives having the general formula [II] | | R⁴ | R⁵ |
|---|---|---|---|
| 13. | daidzein | H | H |
| 14. | glycitein | H | OCH₃ |
| 15. | genistein | OH | H |

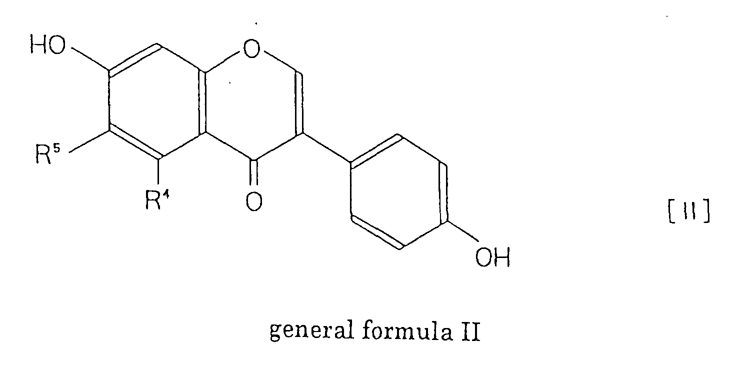

The potential isoflavone derivatives suitably used in the present invention are selected from daidzein, genistein, glycitein, daidzin, genistin, glycitin, 6"-O-malonyldaidzin, 6"-O-malonylgenistin, 6"-O-malonylglycitin, 6"-O-acetyldaidzin, 6"-O-acetylgenistin, 6"-O-acetylglycitin, 6"-O-succinyldaidzin, 6"-O-succinylgenistin, and 6"-O-succinylglycitin; or mixtures thereof. They may be obtained by extraction from soybean or *natto* using a solvent such as hot water, alcohol or the like, followed by an adequate purification procedure. However, most of the isoflavone derivatives contained in such an extract are daidzin and genistin.

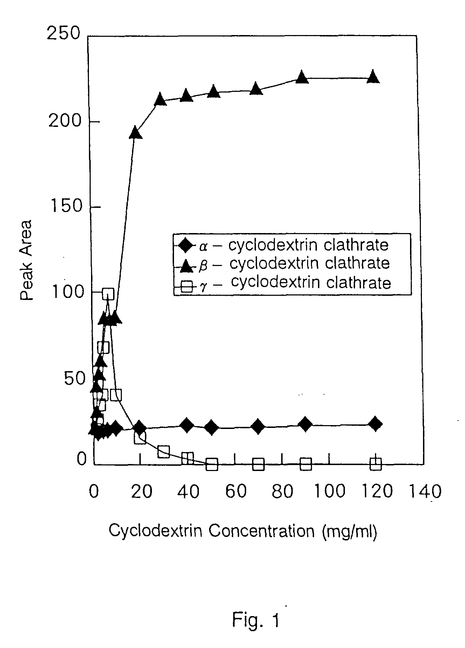

Cyclodextrins are nonreducing maltoligosaccharides, in which 6 - 12 glucose molecules are annularly linked via an α-1,4 glucoside bond, and may be made by a reaction of starch with cyclodextrin glucanotransferase (CGTase) from *Bacillus macerans* and the like. Known general cyclodextrins include *α*-cyclodextrin consisting of six molecules of glucose, β-cyclodextrin consisting of seven molecules of glucose and γ-cyclodextrin consisting of eight molecules of glucose. In the preferred embodiment of the present invention, β-cyclodextrin or γ-cyclodextrin is considered to be a suitable partner of the isoflavone derivatives. Namely, these cyclodextrins may be particularly well clathrated with the isoflavone derivatives, in order to exert the desired effects (see Figures 1 - 8).

These cyclodextrins may be used alone or in combination. Moreover, clathrating with branched or methylated β-cyclodextrin or γ-cyclodextrin having improved solubility may also be effective.

Methods of making the clathrate of the above isoflavone derivatives with the above cyclodextrins are described below.

There are various methods for making clathrates of the isoflavone derivatives with cyclodextrins. One example of these methods is kneading. That is to say, water is added to a cyclodextrin in an amount of about 0.1-6 weight by parts of the cyclodextrin, then the mixture is well kneaded to produce a pasty form, and finally isoflavone derivatives are added thereto followed by sufficient kneading. The dry weight ratio of the isoflavone derivatives to the cyclodextrins may be between 1 : 0.1 and 1 : 50, and preferably, between 1 : 3 and 1 : 10. The kneading time may be from about 30 minutes to 3 hours. As the kneading device, a kneader mixer, ball mill, emulsifier and the like may be used. The paste may be spray dried, dried under reduced pressure, or dried by drum method after clathration.

In the clathrate of the isoflavone derivatives thus obtained, bitterness, acerbity and astringency of the original isoflavone derivatives may be suppressed, and the water solubility of the clathrate may also be improved. Accordingly, they are extremely suitable for application to edible compositions, and may accomplish improvement of absorption rate into the body to enhance the physiological action thereof.

The clathrate of the isoflavone derivatives of the present invention may be mixed in an edible composition to be ingested. The ingestion dose of the clathrate may be varied in accordance with the condition, age or the like of the recipient subject. Usually, 0.1 - 4 mg/kg body weight/day, preferably, 0.2 - 1 mg/kg body weight/day of the isoflavone derivatives dosage is employed. The ingestion can be once per day, otherwise, can be divided into multiple times if necessary.

The edible composition can be of any form, for example, beverage, confectionery, processed foods, seasonings or the like without any limitation.

In order to prepare the edible composition, the ingredients such as excipient, filler, stabilizer, emulsifier, sweetener, flavor, colorant, color fixative and the like, each of which is commonly employed in the art, may be mixed properly, as long as they do not exert undesirable effects to the active component of the edible composition of the present invention.

The non-limiting examples of the present invention are described below to explain the present invention in more detail.

### Examples

### Clathrate formation test:

First, various clathrates were obtained through clathration of several isoflavone derivatives with several cyclodextrins, and the following experiments were carried out to determine water solubility of each clathrate.

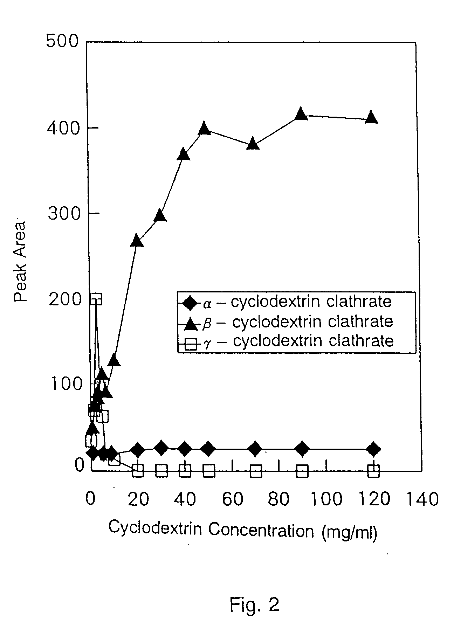

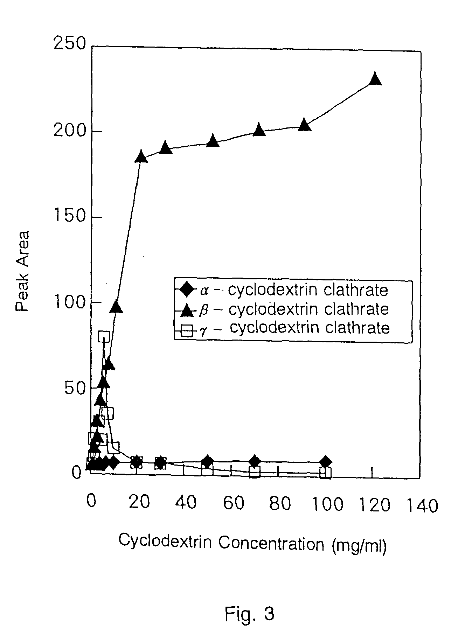

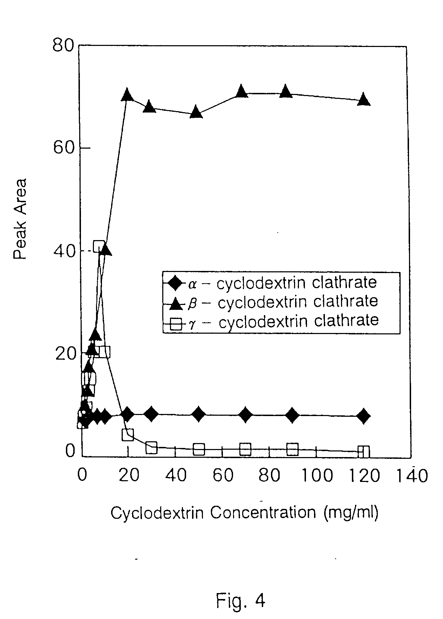

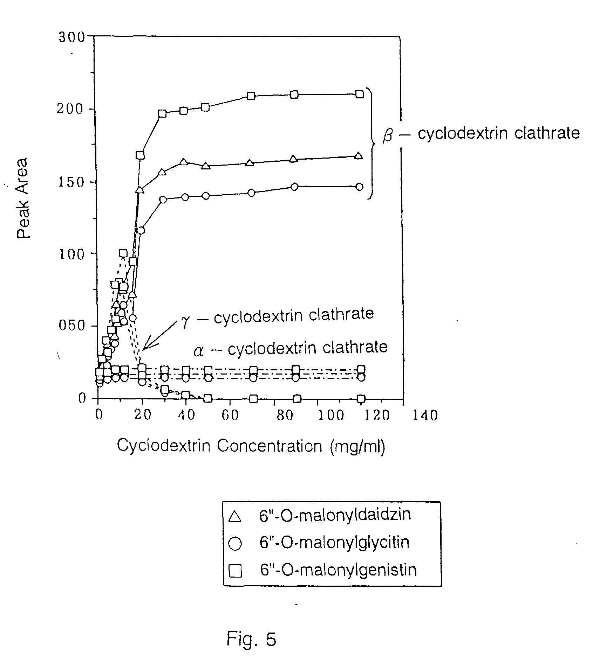

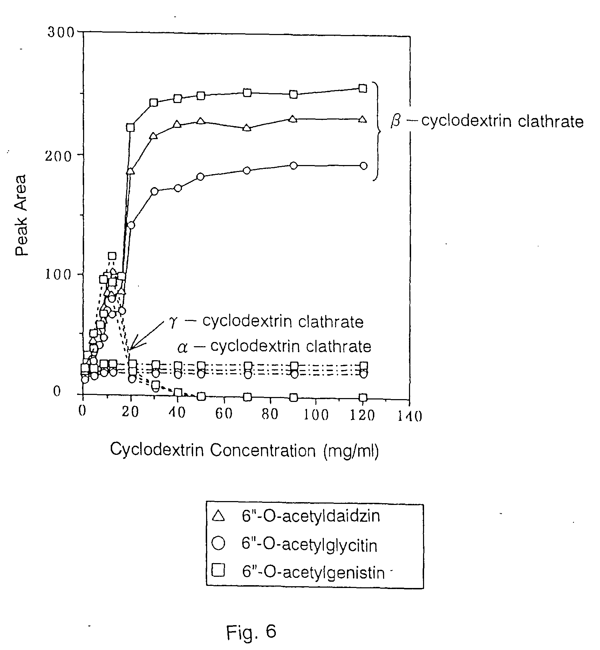

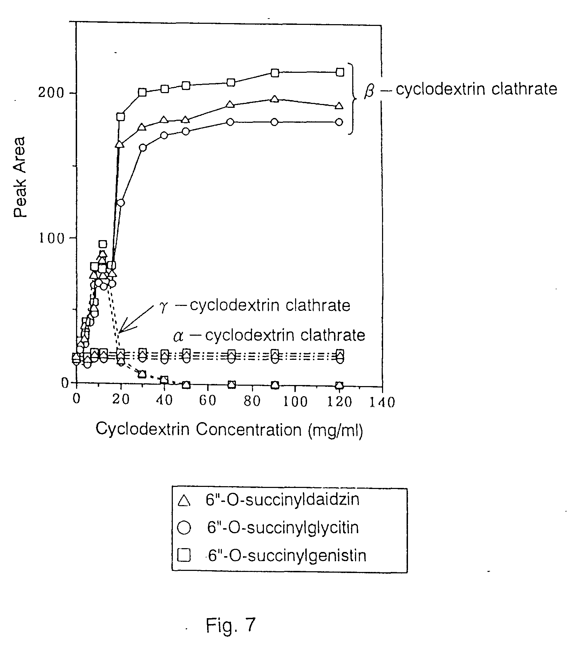

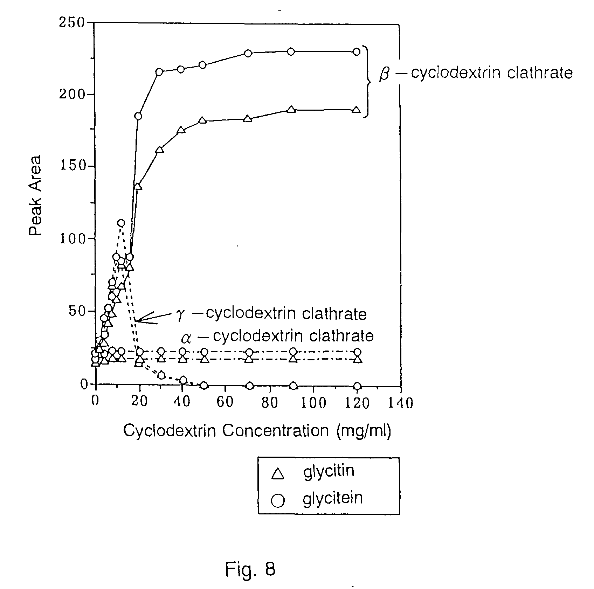

Ten mg of each of fifteen isoflavone derivatives, i.e., daidzin (Fig. 1); genistin (Fig. 2); daidzein (Fig. 3); genistein (Fig. 4); 6"-O-malonyldaidzin, 6"-O-malonylgenistin and 6"-O-malonylglycitin (Fig. 5); 6"-O-acetyldaidzin, 6"-O-acetylgenistin and 6"-O-acetylglycitin (Fig. 6); 6"-O-succinyldaidzin, 6"-O-succinylgenistin and 6"-O-succinylglycitin (Fig. 7); and glycitin and glycitein (Fig. 8) was placed in each test tube respectively with α-,β- or γ-cyclodextrin in an amount shown in Figures 1 to 8 (i.e., 0-1.2 g) together with 10 ml of distilled water, then mixed with stirring overnight at room temperature. Next, each of the solutions was filtered through a glass filter and the filtrate was analyzed using high performance liquid chromatography. High performance liquid chromatography employed was SC-8020 (Toso, JAPAN) equipped with a column YMC pack ODS-AM-303 (ø4.6 x 200 mm, YMC, JAPAN). The solvent system for analysis was a linear gradient from 15% acetonitrile/0.1% acetic acid aqueous solution to 35% acetonitrile/0.1% acetic acid aqueous solution at a flow rate of 1.0 ml/min, and absorption at 254 nm was detected to determine peak areas of the isoflavone derivatives. The results are shown in Figures 1 to 8.

The isoflavone derivatives employed are clathrated with each of the cyclodextrins to form the clathrates. The increase in formation of the clathrate has been shown in Figures 1 to 8, based on the increase in peak areas of respective isoflavone derivatives. Patterns of variation in solubility of respective isoflavone derivatives are similar, and the highest level of clathrate was yielded when β-cyclodextrin was used as a partner, suggesting that best improvement in solubility was accomplished with β-cyclodextrin.

Thereafter, the absorption rate in the body and physiological action of those clathrates of the isoflavone derivatives were examined.

### Example 1: Comparison of absorption rate and physiological action of β-cyclodextrin clathrates with various isoflavone derivatives

To obtain a postmenopausal osteoporosis model, eighty day old Sprague-Dawley rats on day 10 after ovariectomy were grown for 28 days feeding with calcium deficient food (Ca: 0.004%, P: 0.3%), with one group consisting of 5-6 rats. During the rats' growth, several isoflavone derivatives or β-cyclodextrin clathrate thereof (see Table 3) were administered orally in the form of a solution or a suspension in 1% aqueous hydroxypropylcellulose. On the day before the final administration, urine was collected for 24 hours. Blood was collected from postcava at 4 hours after the final administration, and the femurs were removed. The collected blood was separated to obtain serum according to a standard method. In order to determine the amounts of isoflavone derivatives in the obtained samples of urine and serum, the samples were subjected to the reactions for 2 hours at 37°C through adding thereto 50 Units/ml of sulfatase (EC3.1.6.1;SIGMA CHEMICAL Co. LTD.) in 1 ml of acetate buffer (0.1M pH 4.5). Then 1 ml of acetate buffer (0.1 M, pH 4.5) containing β-glucuronidase (200 Units/ml, EC 3.2.1.31, WAKO JYUNYAKU KOGYO KABUSIKI KAISYA) was added to the mixture and incubated for 2 hours at 37°C. After treating it with Sep-Pak (Waters), the incubated solution was analyzed using HPLC as described in the above clathrate formation test. Each of the isoflavone derivative levels in the sample was determined based on the values for daidzein, genistein or glycitein as a standard. The bone density of the femur was calculated based on the wet weight and volume as measured by pycnometer. The results are shown in the Table 3 below.

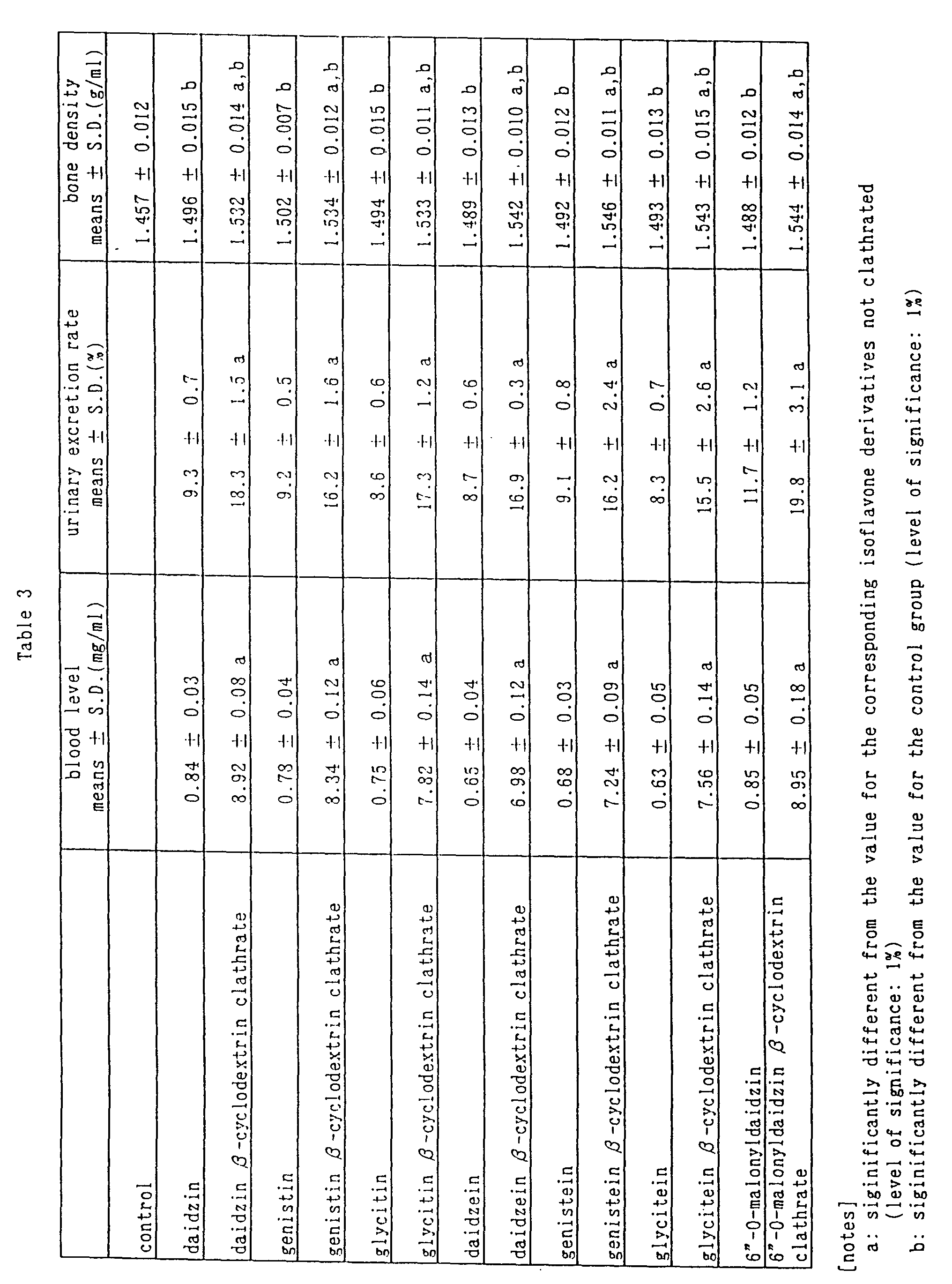

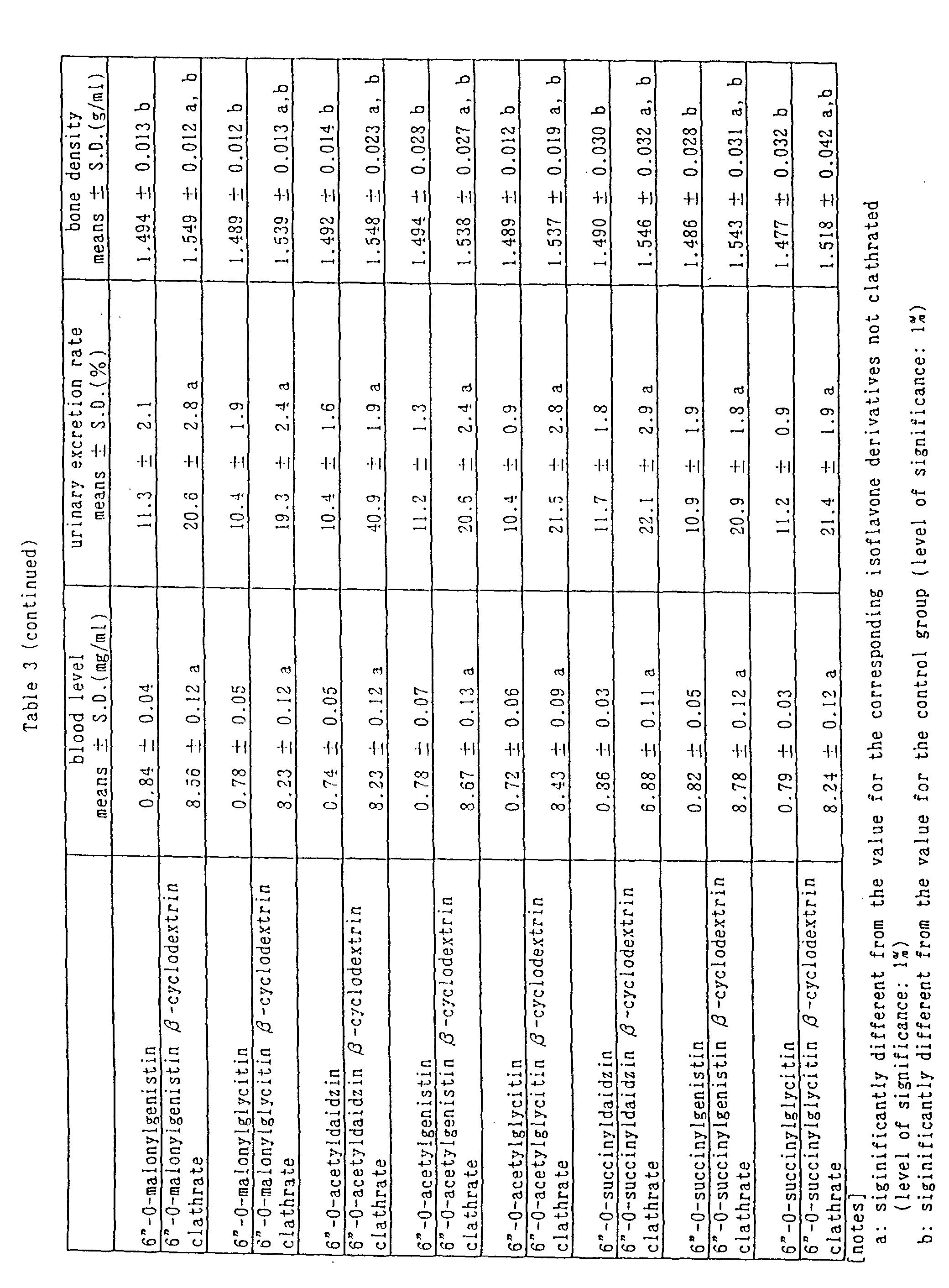

From the results above, each of the concentrations of the isoflavone derivatives in serum samples from the animals at 4 hours after administering the β-cyclodextrin clathrate of the isoflavone derivatives proved to be from approximately 8 to 12 times higher than the samples from the animals administered nonclathrated isoflavone derivatives. Further, urinary excretion level was also raised approximately two-fold. Therefore, clathration of the isoflavone derivatives with cyclodextrins resulted in elevation of the absorption efficiency thereof in the living body. Moreover, in comparison with the control group, bone density of the femur was increased in both groups administered with clathrated and nonclathrated isoflavone derivatives. Thus, potential applicability to prophylaxis or treatment of osteoporosis with those isoflavone derivatives was indicated, and such effect was proved to be more potent in the group administered with the clathrate of the isoflavone derivatives with cyclodextrins.

The clathrate of the present invention was prepared and applicability to the edible compositions thereof was studied as described below.

### Example 2: β-cyclodextrin clathrate of daidzin

Daidzin 2.0 g was dissolved in 300 ml of 50% ethanol at 80°C with 10g of β-cyclodextrin. The solution was mixed with stirring for 2 hours, keeping the temperature around 60-80°C. After the mixture was left at room temperature overnight, it was dried under reduced pressure to yield 11.8 g of powder. The resulting β-cyclodextrin clathrate was evaluated by a panel of ten for bitterness, astringency and roughness.

### Example 3: γ-cyclodextrin clathrate of daidzin

The similar process as described in Example 2 was carried out except that γ-cyclodextrin was used instead of β-cyclodextrin to obtain γ-cyclodextrin clathrate. The resulting γ-cyclodextrin clathrate was similarly evaluated by a panel of ten for bitterness, astringency and roughness.

### Comparative Example 1: α- cyclodextrin clathrate of daidzin

The similar process as described in Example 2 was carried out except that α-cyclodextrin was used instead of β-cyclodextrin to obtain α-cyclodextrin clathrate. The resulting α-cyclodextrin clathrate was similarly evaluated by a panel of ten for bitterness, astringency and roughness.

### Comparative Example 2: Daidzin

Daidzin was evaluated by a panel of ten for bitterness, astringency and roughness.

The results of the evaluations referred to in the Example 2 to Comparative Example 2 are shown in Table 4 below.

**Table 4**

| | clathrate | bitterness | astringency | roughness |
|---|---|---|---|---|
| Example 2 | β-cyclodextrin | - | - | - |
| Example 3 | γ-cyclodextrin | + | + | - |
| Comparative Example 1 | α-cyclodextrin | +++ | +++ | +++ |
| Comparative Example 2 | - | ++++ | ++++ | ++++ |
| [notes for tasting] - : none + : slight ++ : middle +++ : strong ++++ : very strong | | | | |

Apparently from the results shown in Table 4 above, it is evident that the bitterness, astringency and roughness of daidzin are suppressed when the isoflavone derivatives are clathrated with β-cyclodextrin or γ-cyclodextrin. Further, it is suggested that suppression of the bitterness, astringency or roughness of daidzin is more remarkable when the isoflavone derivatives are clathrated with β-cyclodextrin.

### Example 4: Clathrate of isoflavone derivatives with α-, β-, or γ-cyclodextrin

The similar process as described in Examples 2, 3 and Comparative Example 1 was carried out to obtain clathrates of various isoflavone derivatives with α-, β-, or γ-cyclodextrin. The resulting clathrates were evaluated by a panel often for bitterness, astringency and roughness. The results of evaluations are shown in Table 5 below.

**Table 5**

| isoflavone derivatives | clathrate | bitterness | astringency | roughness |
|---|---|---|---|---|
| glycitin | β-cyclodextrin | - | - | - |
| | γ-cyclodextrin | + | + | - |
| | α-cyclodextrin | +++ | +++ | +++ |
| genistin | β-cyclodextrin | - | - | - |
| | γ-cyclodextrin | + | + | - |
| | α-cyclodextrin | +++ | +++ | +++ |
| 6"-0-acetyldaidzin | β-cyclodextrin | - | - | - |
| | γ-cyclodextrin | + | + | - |
| | α-cyclodextrin | +++ | +++ | +++ |
| 6"-0-acetylglycitin | β-cyclodextrin | - | - | - |
| | γ-cyclodextrin | + | + | - |
| | α-cyclodextrin | +++ | + + + | +++ |
| 6"-0-acetylgenistin | β-cyclodextrin | - | - | - |
| | γ-cyclodextrin | + | + | - |
| | α-cyclodextrin | + + + | + + + | + + + |
| 6"-0-malonyldaidzin | β-cyclodextrin | - | - | - |
| | γ-cyclodextrin | + | + | - |
| | α-cyclodextrin | + + + | + + + | + + + |
| 6"-0-malonylglycitin | β-cyclodextrin | - | - | - |
| | γ-cyclodextrin | + | + | - |
| | α-cyclodextrin | +++ | +++ | +++ |
| 6"-0-malonylgenistin | β-cyclodextrin | - | - | - |
| | γ-cyclodextrin | + | + | - |
| | α-cyclodextrin | +++ | +++ | +++ |
| 6"-0-succinyldaidzin | β-cyclodextrin | - | - | - |
| | γ-cyclodextrin | + | + | - |
| | α-cyclodextrin | +++ | +++ | +++ |
| 6"-0-succinylglycitin | β-cyclodextrin | - | - | - |
| | γ-cyclodextrin | + | + | - |
| | α-cyclodextrin | +++ | +++ | +++ |
| 6"-0-succinylgenistin | β-cyclodextrin | - | - | - |
| | γ-cyclodextrin | + | + | - |
| | α-cyclodextrin | +++ | +++ | +++ |
| daidzein | β-cyclodextrin | - | - | - |
| | γ-cyclodextrin | + | + | - |
| | α-cyclodextrin | +++ | +++ | +++ |
| glycitein | β-cyclodextrin | - | - | - |
| | γ-cyclodextrin | + | + | - |
| | α-cyclodextrin | +++ | +++ | +++ |
| genistein | β-cyclodextrin | - | - | - |
| | γ-cyclodextrin | + | + | - |
| | α-cyclodextrin | +++ | +++ | +++ |
| [notes for tasting] - : no + : slight ++ : middle +++ : strong ++++ : very strong | | | | |

As is clear from Table 5, the bitterness, astringency and roughness of any isoflavone derivatives are suppressed when they were clathrated with β-or γ-cyclodextrin.

### Example 5: Beverage containing the cyclodextrin clathrate of isoflavone derivative

One hundred and twenty mg of the daidzin clathrated with β-cyclodextrin as described in Example 2 was dissolved in 90 ml of water. Nine grams of fruit juice, 5 g of granulated sugar, 0.5 g of citric acid and 0.1 g of flavor were added thereto, then mixed with stirring. The mixture was successively sterilized for an instant at 122-138°C, bottled in a 100 cc glass bottle to make beverage containing the clathrate of daidzin. The resulting beverage was evaluated by a panel of ten for bitterness, astringency and roughness.

### Comparative Example 3: Beverage containing isoflavone derivative

A process similar to Example 5 was carried out except that 20 mg of daidzin powder was used instead of the clathrate described in Example 2 to prepare a beverage containing daidzin. The resulting beverage was evaluated by a panel of ten for bitterness, astringency and roughness.

The results of the evaluations referred to in the Example 5 and Comparative Example 3 are shown in Table 6 below.

**Table 6**

| | clathrate | bitterness | astringency | roughness |
|---|---|---|---|---|
| Example 5 | β-cyclodextrin | - | - | - |
| Comparative Example 3 | - | ++++ | ++++ | +++ |
| [notes for tasting] - : none + : slight ++ : middle +++ : strong ++++ : very strong | | | | |

It is apparent from Table 6 that the beverage containing powdered daidzin had bitterness, astringency and roughness (Comparative Example 3), while the beverage containing clathrated daidzin with β-cyclodextrin did not have bitterness, astringency and roughness at all (Example 5). Moreover, precipitate was found immediately subsequent to completion of preparing the beverage according to Comparative Example 3. On the contrary, no precipitate was found even after 3 months subsequent to preparation of the beverage according to Example 4. It was therefore concluded that higher water solubility could be achieved when the isoflavone derivatives were clathrated.

### Example 6: Cookies containing the cyclodextrin clathrate of isoflavone derivative

Eighty grams of butter and 0.1 g of salt were mixed to be creamy using a whip mixer, then 40 g of confectioner's sugar was added and ground with rubbing. After an egg which had been broken and beaten was added thereto and the resulting mixture was mixed, then, sift flour 100 g including 480 mg of the clathrate of daidzin with β-cyclodextrin (see Example 2) was also added and mixed. Thereafter, the resulting dough was rolled to 4 mm thickness using a roller, then cut with a dough cutter, baked for 12-13 minutes at approximately 160°C - 170°C in an oven.

The cookies thus made had neither bitterness nor astringency, and tasted very good.

### Example 7: Clathrate of crude isoflavone derivatives with β-cyclodextrin

Twenty ml of water was added to 10.0 g of β-cyclodextrin, and the mixture was kneaded for 30 minutes to be pasty. Then, 2.0 g of crude isoflavone derivatives (isoflavone derivatives content: 70%) obtained by extraction from soybean with hot water followed by purification of the extract using synthetic absorbent was added to the paste, and further kneading was conducted for 2 hours. Thereafter, the paste was dried under reduced pressure, to yield 11.6 g of powder. The clathrate with β-cyclodextrin thus obtained was evaluated for bitterness, astringency and roughness by a panel of ten.

### Example 8: Clathrate of crude isoflavone derivatives with γ-cyclodextrin

The similar process as described in Example 7 was carried out except that γ-cyclodextrin was used instead of β-cyclodextrin to obtain γ-cyclodextrin clathrate. The resulting γ-cyclodextrin clathrate was similarly evaluated by a panel of ten for bitterness, astringency and roughness.

### Comparative Example 4: Clathrate of crude isoflavone derivatives with α-cyclodextrin

The similar process as described in Example 7 was carried out except that α-cyclodextrin was used instead of β-cyclodextrin to obtain α-cyclodextrin clathrate. The resulting α-cyclodextrin clathrate was similarly evaluated by a panel of ten for bitterness, astringency and roughness.

### Comparative Example 5: Crude isoflavone derivatives

The crude isoflavone derivatives described in Example 7 was evaluated by a panel often for bitterness, astringency and roughness.

The evaluations referred to in the Example 7 to Comparative Example 5 are shown in the Table 7 below.

**Table 7**

| | clathrate | bitterness | astringency | roughness |
|---|---|---|---|---|
| Example 7 | β-cyclodextrin | - | - | - |
| Example 8 | γ-cyclodextrin | + | + | - |
| Comparative Example 4 | α-cyclodextrin | +++ | +++ | +++ |
| Comparative Example 5 | - | ++++ | ++++ | ++++ |
| [notes for tasting] - : none + : slight +++ : strong ++++ : very strong | | | | |

As shown in Table 7, it is suggested that bitterness, astringency and roughness of the crude isoflavone derivatives could be suppressed through clathration with β-cyclodextrin or γ-cyclodextrin. Especially, when they are clathrated with β-cyclodextrin, the bitterness, the astringency and the roughness was evidently suppressed. These results are similar to the above described results in Examples 2 and 3, and Comparative Examples 1 and 2.

### Example 9: Jelly containing cyclodextrin clathrate of crude isoflavone derivatives

To 150 ml of water, 360 mg of the clathrate of the crude isoflavone derivatives with β-cyclodextrin obtained in accordance with Example 7 was dissolved. Then, 50 g of fruit juice and granulated sugar were added to the solution. After 2.5 g of agar was added, the mixture was heated to 90°C with stirring thoroughly to melt the agar. The heated solution was poured into the 50 ml cups made of plastics, and cooled to 5-10°C to harden. Thus, a jelly containing crude isoflavone derivatives was prepared.

The jelly was evaluated for bitterness, astringency and roughness by a panel of ten. The results of the evaluations are shown in Table 8. The jelly made in such a manner had no bitterness, astringency or roughness. Moreover, the jelly was very tasty, and appeared clear without any precipitate.

### Comparative Example 6: Jelly containing crude isoflavone derivatives

A process which is similar to Example 9 was performed except that 60mg of the crude isoflavone derivatives described in Example 7 were used instead of the β-cyclodextrin clathrate to prepare jelly containing the isoflavone derivatives. The resulting jelly was evaluated by a panel of ten for bitterness, astringency and roughness. The results are shown in Table 8.

**Table 8**

| | clathrate | bitterness | astringency | roughness |
|---|---|---|---|---|
| Example 9 | β-cyclodextrin | - | - | - |
| Comparative Example 6 | - | ++++ | ++++ | +++ |
| [notes for tasting] - : none +++ : strong ++++ : very strong | | | | |

It is apparent from Table 8 that the jelly containing the crude isoflavone derivatives which were not clathrated with (3-cyclodextrin displayed bitterness, astringency and roughness. On the other hand, the jelly containing the crude isoflavone derivatives clathrated with β-cyclodextrin did not display any bitterness, astringency or roughness. Additionally, no precipitate could be found in the jelly of Example 9 while some could be found in that of Comparative Example 6. Therefore, solubility of the crude isoflavone derivatives are also improved through clathration with the cyclodextrins.

According to the present invention, water solubility of isoflavone derivatives can be improved and, suppression of bitterness and astringency can be achieved through clathrating the isoflavone derivatives with cyclodextrins.

In accordance with the present invention, the isoflavone derivatives can therefore be applied to edible compositions. Namely, edible compositions containing the isoflavone derivatives having higher water solubility are prepared without undesirable taste.

Accordingly, the present invention can facilitate the oral ingestion and absorption into the body of isoflavone derivatives. Thus, the beneficial effects of the isoflavone derivatives can be obtained in the human body, contributing to the maintenance and promotion of good health.

## Claims

1. A clathrate comprising an isoflavone derivative with β-cyclodextrin and/or γ-cyclodextrin, wherein the isoflavone derivative is selected from daidzein, genistein, glycitein, daidzin, genistin, glycitin, 6"-O-malonyldaidzin, 6"-O-malonylgenistin, 6"-O-malonylglycitin, 6"-O-acetyldaidzin, 6"-O-acetylgenistin, 6"-O-acetylglycitin, 6"-O-succinyldaidzin, 6"-O-succinylgenistin, and 6-O-succinylglycitin; or a mixture thereof.

2. A clathrate according to claim 1 wherein said clathrate is obtained by clathration in the ratio of said β-cyclodextrin and/or γ-cyclodextrin : isoflavone derivatives of between 0.1:1 and 50:1.

3. An edible composition comprising the clathrate according to claim 1 or claim 2.

4. A clathrate or composition according to any one of claims 1 to 3 for use in the prophylaxis or treatment of osteoporosis.

## Patentansprüche

1. Clathrat, das ein Isoflavon-Derivat mit β-Cyclodextrin und/oder γ-Cyclodextrin aufweist, wobei das Isoflavon-Derivat aus Daidzein, Genistein, Glycitein, Daidzin, Genistin, Glycitin, 6"-O-Malonyldaidzin, 6"-O-Malonylgenistin, 6"-O-Malonylglycitin, 6"-O-Acetyldaidzin, 6"-O-Acetylgenistin, 6"-O-Acetylglycitin, 6"-O-Succinyldaidzin, 6"-O-Succinylgenistin und 6-O-Succinylglycitin ausgewählt ist, oder eine Mischung davon ist.

2. Clathrat nach Anspruch 1, wobei das Clathrat durch Clathratbildung in dem Verhältnis β-Cyclodextrin und/oder γ-Cyclodextrin : Isoflavon-Derivate von zwischen 0,1:1 und 50:1 erhalten wird.

3. Eßbare Zusammensetzung, die das Clathrat nach Anspruch 1 oder Anspruch 2 aufweist.

4. Clathrat oder Zusammensetzung nach einem der Ansprüche 1 bis 3 zur Verwendung bei der Prophylaxe oder Behandlung von Osteoporose.

## Revendications

1. Clathrate comprenant un dérivé d'isoflavone avec une β-cyclodextrine et/ou une γ-cyclodextrine, dans lequel le dérivé d'isoflavone est choisi parmi la daidzéine, la génistéine, la glycitéine, la daidzine, la génistine, la glycitine, la 6"-O-malonyldaidzine, la 6"-O-malonylgénistine, la 6"-O-malonyglycitine, la 6"-O-acétyldaidzine, la 6"-O-acétylgénistine, la 6"-O-acétylglycitine, la 6"-O-succinyldaidzine, la 6"-O-succinylgénistine, et la 6-O-succinylglycitine ou un mélange de ceux-ci.

2. Clathrate selon la revendication 1 dans lequel ledit clathrate est obtenu par formation d'un clathrate, le rapport dudit β-cyclodextrine et/ou γ-cyclodextrine : dérivés d'isoflavone étant compris entre 0,1:1 et 50:1.

3. Composition comestible comprenant le clathrate selon la revendication 1 ou 2.

4. Clathrate ou composition selon l'une quelconque des revendications 1 à 3 pour l'utilisation dans la prophylaxie ou le traitement de l'ostéoporose.
